# EUROPÄISCHE PATENTSCHRIFT

(11) **EP 0 743 309 B1**
(45) Veröffentlichungstag und Bekanntmachung des Hinweises auf die Patenterteilung: **10.09.2003**
(21) Anmeldenummer: 96810297.0
(22) Anmeldetag: 09.05.1996
(51) Int. Cl.: C07D 251/24, A61K 7/42, C08K 5/3492

(54) **o-Hydroxyphenyl-s-triazine als UV-Stabilisatoren**
o-Hydroxyphenyl-s-triazines as UV stabilisers
o-Hydroxyphényl-s-triazines comme UV-stabilisateurs

(30) Priorität: 18.05.1995 CH 147995
(43) Veröffentlichungstag der Anmeldung: 20.11.1996
(73) Patentinhaber: Ciba Specialty Chemicals Holding Inc., 4057 Basel (CH)
(72) Erfinder: Luther, Helmut, Dr., 79639 Grenzach-Wyhlen (DE); Hüglin, Dietmar, Dr., 79104 Freiburg (DE); Herzog, Bernd, Dr., 79639 Grenzach-Wyhlen (DE)

(56) Entgegenhaltungen:
- EP-A- 0 648 753
- EP-A- 0 648 754
- EP-A- 0 649 841
- EP-A- 0 654 469
- DE-A- 1 469 811
- GB-A- 2 286 774

## Beschreibung

Die vorliegende Erfindung betrifft o-Hydroxyphenyl-s-triazine, die mindestens zwei Alkoxyphenyl-Substituenten enthalten, Herstellungsverfahren für diese Verbindungen, die Verwendung zum photochemischen und thermischen Stabilisieren von gefärbten und ungefärbten Polyesterfasermaterialien, die Verwendung dieser Verbindungen als Stabilisatoren für organische Polymere, das mit diesen Verbindungen stabilisierte Polymer sowie die Verwendung dieser Verbindungen als kosmetische Mittel.

Die EP-A-0,648,754 offenbart die Herstellung von gewissen Hydroxyphenyl-1,3,5-triazinen. die sich als UV-Absorber für organische Materialien eignen.

Die EP-A-0,648,753 offenbart ein Verfahren zur Herstellung von gewissen Hydroxyphenyl-1,3,5-triazinen. Die Verbindungen finden Verwendung als UV-Absorber oder als Ausgangsprodukte zur Herstellung von UV-Absorbern.

Die DE-A-1,469,811 offenbart die Stabilisierung von polymeren Materialien gegen die schädlichen Einflüsse von UV-Licht durch Hydroxyphenyl-1,3,5-triazine.

Die EP-A-0,649,841 offenbart ein Herstellungsverfahren für hydroxydiphenylsubstituierten 1,3,5-Triazinen. Diese Triazine unterscheiden sich von den erfindungsgemässen.

Die EP-A-0,654,469 offenbart o-Hydroxyphenyl-s-triazine, die mindestens 2 Alkoxyphenylsubstituenten enthalten. Diese Triazine unterscheiden sich von den erfindungsgemässen.

Die GB-A-2,286,774 offenbart mikronisierte, unlösliche organische UV-Absorber, die geeignet sind für kosmetische und pharmazeutische Anwendungen.

Die neuen o-Hydroxyphenyl-s-triazine entsprechen der Formel 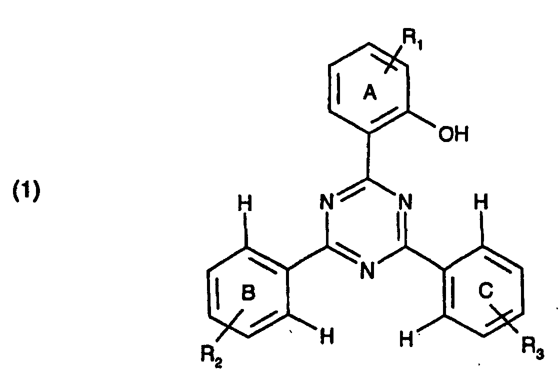 worin
- R₁: Wasserstoff, und
- R₂ und R₃: 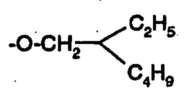
bedeuten.

Die Verbindungen der Formel (1) lässt sich nach verschiedenen Verfahren herstellen.

Beispielsweise lässt sich die Verbindung der Formel (1) in einem einstufigen Verfahren durch Umsetzung einer Salicylverbindung mit einer Benzamidinverbindung herstellen. Das Herstellungsverfahren ist dadurch gekennzeichnet, dass man eine Salicylverbindung der Formel 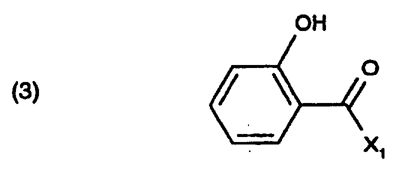 mit einer Benzamidinverbindung der Formel 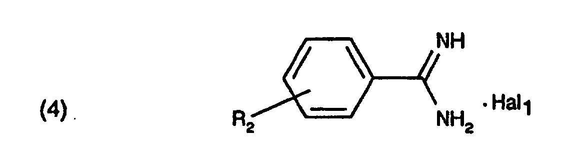 zur Triazinverbindung der Formel (1) umsetzt, wobei
- R₁ und R₂,: die in Formel (1) angegebene Bedeutung haben
- X₁: Halogen oder -OR₄,
- R₄: C₁-C₃-Alkyl und
- Hal₁: Halogen bedeuten.

Bei den Ausgangsverbindungen der Formel (3) handelt es sich um gegebenenfalls substituierte Salicylsäureester oder Salicylsäurehalogenide, wie z.B. Salicylsäuremethyl-, Salicylsäureethyl- oder Salicylsäurepropylester, bzw. Salicylsäurechlorid oder -bromid, die im Phenylrest, entsprechend der Bedeutung von A, durch weitere Reste substituiert sein können.

Bei dem erfindungsgemässen Verfahren können die Ausgangsverbindungen der Formeln (3) und (4) in verschiedenen molaren Verhältnissen eingesetzt werden.

Vorzugsweise betragen die Molverhältnisse der Verbindung der Formel (3) zur Verbindung der Formel (4) 1:10 bis 10:1.

Ist die Ausgangsverbindung der Formel (3) ein Salicylsäurehalogenid (X₁=Halgen), beträgt das molare Verhältnis der Verbindung der Formel (3) zur Verbindung der Formel (4) vorzugsweise 1:3 bis 1:2.

Wird als Ausgangsverbindung der Formel (3) ein Salicylsäureester (X₁= -OR₄) eingesetzt, beträgt das molare Verhältnis der Verbindung der Formel (3) zur Verbindung der Formel (4) vorzugsweise 2:1 bis 1:2.

Als Benzamidinverbindungen der Formel (4) kommen das Benzamidinhydrobromid und vorzugsweise das -chlorid, die im Phenylrest entsprechend der Bedeutung von B weiter substituiert sein können, in Betracht. Diese Verbindungen werden normalerweise als Festprodukte mit einem Gehalt an Aktivsubstanz von ca. 90-95% eingesetzt.

Verwendet man als Ausgangsverbindung der Formel (3) ein Salicylsäurehalogenid (X₁=Halogen), wird in der Regel zur Neutralisation der bei der Reaktion entstehenden Säure mindestens die berechnete Menge einer Base zugegeben. Als Basen können sowohl organische als auch anorganische Verbindungen verwendet werden, wie z.B. Alkalihydroxid, insbesondere Natron- oder Kalilauge; wässrige Ammoniaklösung; Ammoniakgas; Alkalicarbonat, insbesondere Natrium- oder Kaliumcarbonat; Natriumacetat; tert. Amine wie Pyridin oder Trialkylamine, insbesondere Triethylamin, N,N-Dimethylcyclohexylamin, N,N-Dimethylanilin; Alkalialkylate, insbesondere Natrium- und Kaliummethylat oder Kaliumtert.butylat.

Das erfindungsgemässe Verfahren wird in der Regel so durchgeführt, dass man die Salicylund die Benzamidinverbindung in einem inerten Lösungsmittel vorlegt.

Als inerte Lösungsmittel kommen dabei aliphatische Kohlenwasserstoffe und deren Mischungen, wie z.B. Cyclohexan oder aromatische Kohlenwasserstoffe wie Toluol, oder Dimethylacetamid oder Mischungen dieser Lösungsmittel in Betracht.

Verwendet man als Ausgangsverbindung der Formel (3) ein Salicylsäurehalogenid (X₁=Hal), kann noch ein weiteres, in der Regel polares Lösungsmittel, wie z.B. Acetonitril oder Dioxan zugesetzt werden.

Die Reaktionszeit für das erfindungsgemässe Verfahren beträgt im allgemeinen 2 bis 30 Stunden. Je nachdem, ob man als Ausgangsverbindung der Formel (3) ein Salicylsäurehalogenid (X₁=Hal) oder ein Salicylsäureester (X₁=-OR₄) einsetzt, können die Reaktionszeiten variieren. Verwendet man einen Salicylsäureester, beträgt die Reaktionszeit vorzugsweise 4 bis 30 Stunden, insbesondere 18 bis 22 Stunden. Verwendet man ein Salicylsäurehalogenid, sind die Reaktionszeiten etwas kürzer. Sie betragen vorzugsweise 2 bis 20 Stunden, insbesondere 4 bis 8 Stunden.

Die Reaktionen verlaufen in der Regel leicht exotherm ab. Allerdings sollte eine Reaktionstemperatur von 95°C nicht überschritten werden, da bei höheren Temperaturen Nebenprodukte entstehen können, wie beispielsweise Nitrilverbindungen aus den Benzamidinen. In der Praxis wird die Reaktion in einem Temperaturbereich von 60 bis 95°C, vorzugsweise 80 bis 95°C durchgeführt.

Weiterhin können die erfindungsgemässen Verbindungen der Formel (1) durch Dehydrierung einer Dihydrotriazinverbindung der Formel 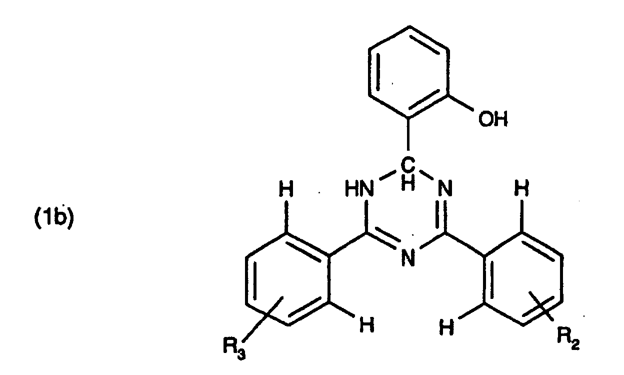 hergestellt werden. R₁, R₂ und R₃ haben dabei die in Formel (1) angegebene Bedeutung.

Als Dehydrierungsmittel wird in der Regel Chloranil eingesetzt. Die Dehydrierung von Dihydrotriazinverbindungen zu 1,3,5-Triazinen mit Hilfe von Chloranil ist z.B. aus der Khim. Geteritsikl. Soedin. (2), S. 350-353 (1969) bekannt.

Die Herstellung der Ausgangsverbindungen der Formel (1b) erfolgt in an sich bekannter Weise durch Umsetzung von 2 Mol einer entsprechenden Benzamidinhydrohalogenidverbindung mit einem Mol einer entsprechenden α-Hydroxybenzaldehydverbindung.

Ein weiterer Weg zur Herstellung der Triazinverbindungen der Formel (1) besteht in der Umsetzung einer Monochlortriazinverbindung der Formel 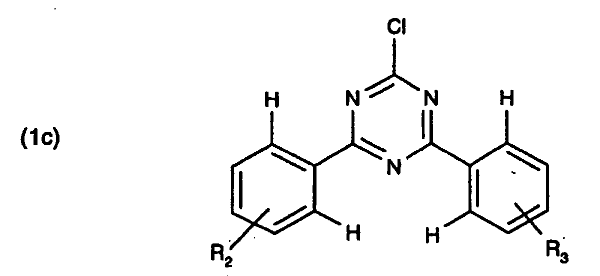 mit einer α-Hydroxyphenylverbindung der Formel 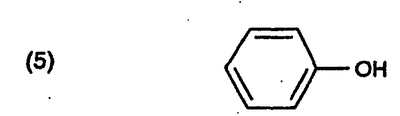 in Gegenwart einerLewis-Säure, insbesondere Aluminiumchlorid.

R₁, R₂, und R₃, haben dabei die in Formel (1) angegebene Bedeutung. Diese Reaktion ist zum Beispiel aus der J. Am. Chem. Soc. 73(7) (1951) bekannt.

Die Ausgangsverbindungen der Formel (1c) lassen sich in an sich bekannter Weise z.B. durch Umsetzung von Cyanurchlorid und den entsprechenden Phenylmagnesiumbromidverbindungen In einer Grignardreaktion herstellen. Diese Reaktion ist z.B. durch Hirt et al., Helv. Chim. Acta, 33, 1368 (1950) bekannt.

Weiterhin lassen sich die erfindungsgemässen Triazinverbindungen durch Umsetzung einer Areno-Oxazinonverbindung der Formel 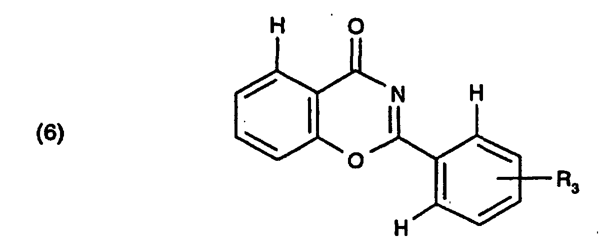 mit einer Benzamidinverbindung der Formel (4) herstellen. R₁, R₂, und R₃ haben dabei die angegebenen Bedeutungen.

Die Areno-Oxazinonverbindungen der Formel (6) sowie die Herstellung dieser Verbindungen sind bekannt, z.B. aus der GB-B-1,155,506.

Die Verbindungen der Formel (1) eignen sich als UV-Stabilisatoren, d.h. zum Schützen von ultraviolett empfindlichen organischen Materialien, insbesondere textilen Fasermaterialien vor der schädigenden Einwirkung von Ultraviolettstrahlung.

Die Triazinverbindungen der Formel (1) sind in Wasser schwerlöslich und werden daher in dispergierter Form appliziert. Dazu werden sie mit einem entsprechenden Dispergator mit Hilfe von z.B. einer Quarzkugelmühle oder einem Schnellrührgerät auf eine Feinheit von etwa 1-2 µm gemahlen.

Als Dispergatoren für die Verbindungen der Formel (1) kommen z.B. in Betracht:
- saure Ester oder deren Salze von Alkylenoxidaddukten, wie z.B. saure Ester oder deren Salze eines Polyadduktes von 4 bis 40 Mol Ethylenoxid an 1 Mol eines Phenols; oder Phosphorsäureester der Addukte von 6 bis 30 Mol Ethylenoxid an 1 Mol 4-Nonylphenol, 1 Mol Dinonylphenol oder besonders an 1 Mol von Verbindungen, die durch Anlagerung von 1 bis 3 Mol von gegebenenfalls substituierten Styrolen an 1 Mol Phenol hergestellt werden,
- Polystyrolsulfonate,
- Fettsäuretauride,
- alkylierte Diphenyloxid-mono- oder -di-sulfonate,
- Sulfonate von Polycarbonsäureestern,
- mit einer organischen Dicarbonsäure, oder einer anorganischen mehrbasischen Säure in einen sauren Ester übergeführte Anlagerungsprodukte von 1 bis 60, vorzugsweise 2 bis 30 Mol Ethylenoxid und/oder Propylenoxid an Fettamine, Fettamide, Fettsäuren oder Fettalkohole mit je 8 bis 22 Kohlenstoffatomen oder an drei- bis sechswertige Alkanole mit 3 bis 6 Kohlenstoffatomen,
- Ligninsulfonate, und ganz besonders
- Formaldehyd-Kondensationsprodukte wie z.B. Kondensationsprodukte von Ligninsulfonaten und/oder Phenol und Formaldehyd, Kondensationsprodukte von Formaldehyd mit aromatischen Sulfonsäuren, wie Kondensationsprodukte von Ditolylethersulfonaten und Formaldehyd, Kondensationsprodukte von Naphthalinsulfonsäure und/oder Naphthol- oder Naphthylaminsulfonsäuren mit Formaldehyd, Kondensationsprodukte von Phenolsulfonsäuren und/oder sulfoniertem Dihydroxydiphenylsulfon und Phenolen bzw. Kresolen mit Formaldehyd und/oder Harnstoff sowie Kondensationsprodukte von Diphenyloxid-disulfonsäure-Derivaten mit Formaldehyd.

Die Verbindungen der Formel (1) können vorteilhaft als Stabilisatoren für organische Polymere gegen deren Schädigung durch Licht, Sauerstoff und Wärme verwendet werden. Beispiele für solche zu stabilisierenden Polymere sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, Vlb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen la, lla und/oder IIIa sind. Die Aktivatoren können beispielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-lsobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (lonomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und - methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat--Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polpropylenoxid oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genannten Polyamide mit Polyolefinen, Olefin-Copolymeren, lonomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gesättigter und ungesättigter Dicarbonsäuren mit mehrwertigen Alkoholen, sowie Vinylverbindungen als Vemetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Hamstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Besonders bevorzugt ist die Verwendung der erfindungsgemäßen Verbindungen als Stabilisatoren in Lacken aller Art. Dies bedeutet auch ein Verfahren gemäß obiger Beschreibung, worin das organische Polymer ein Bindemittel für einen Lack ist. Bei den Lacken kann es sich um pigmentierte oder unpigmentierte Lacke oder Metalleffektlacke handeln. Sie können ein organisches Lösungsmittel enthalten oder lösungsmittelfrei sein oder wässrige Lacke sein.

Die Lacke können als Bindemittel mindestens eines der vorhin aufgeführten Polymeren enthalten. Beispiele für Lacke mit speziellen Bindemitteln sind die folgenden:
1. Lacke auf Basis von kalt- oder heiß-vernetzbaren Alkyd-, Acrylat-, Polyester-, Epoxidoder Melaminharzen oder Mischungen solcher Harze, gegebenenfalls mit Zusatz eines sauren Härtungskatalysators;
2. Zweikomponenten-Polyurethanlacke auf Basis von hydroxylgruppenhaltigen Acrylat-, Polyester- oder Polyetherharzen und aliphatischen oder aroma- tischen Polyisocyanaten;
3. Einkomponenten-Polyurethanlacke auf Basis von blockierten Polyisocyanaten, die während des Einbrennens deblockiert werden;
4. Zweikomponentenlacke auf Basis von (Poly)ketiminen und aliphatischen oder aromatischen Polyisocyanaten;
5. Zweikomponentenlacke auf Basis von (Poly)ketiminen und einem ungesättigten Acrylatharz oder einem Polyacetoacetatharz oder einem Methacrylamidoglykolat-methylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten;
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis siloxanmodifizierter oder fluormodifizierter Acrylatharze.

Die Lacke können auch strahlenhärtbare Lacke sein. In diesem Fall besteht das Bindemittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemäßen Stabilisatoren vorzugsweise der unpigmentierten obersten Schicht zugesetzt werden.

Die Lacke können auf die Substrate (Metall, Plastik, Holz etc.) nach den üblichen Verfahren aufgebracht werden, beispielsweise durch Streichen, Besprühen, Gießen, Tauchen oder Elektrophorese.

Die Menge des zugesetzten Stabilisators der Formel (1) hängt vom jeweiligen Substrat und dessen Verwendungszweck ab. Im allgemeinen genügen Mengen von 0,01 bis 5Gew.-%, bevorzugt verwendet man 0,05 bis 3Gew.-%, bezogen auf das zu stabilisierende Polymer. Erfindungsgemäß sind daher insbesondere Polymere enthaltend 0,01 bis 5Gew.-%, vor allem 0,05 bis 3Gew.-%, mindestens einer Verbindung der Formel (1) geeignet.

In bestimmten Fällen kann es von Vorteil sein, zwei oder mehrere der Verbindungen der Formel (1) zu verwenden. Außerdem können ein oder mehrere andere Stabilisatoren und/oder sonstigen Zusatzstoffe mitverwendet werden, wie z.B. die folgenden Typen von Verbindungen:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tertbutyl-4-methoxymethylphenol, 2,6-Di-nonyl-4-methylphenol, 2,4-Dimethyl-6-(1'-methylundec-1'-yl)-phenol, 2,4-Dimethyl-6-(1'-methyl-heptadec-1'-yl)-phenol, 2,4-Dimethyl-6-(1'methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol; 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.
   1.4. Hydroxylierte Thiodiphenylether, z.B. 2,2'-Thio-bis-(6-tert-butyl-4-methylphenol), 2,2'--Thio-bis-(4-octylphenol), 4,4'-Thio-bis-(6-tert-butyl-3-methylphenol), 4,4'-Thio-bis-(6-tertbutyl-2-methylphenol), 4,4'-Thio-bis-(3,6-di-sec.-amylphenol), 4,4'-Bis-(2,6-dimethyl-4-hydroxyphenyl)-disulfid.
   1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tertbutylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methyl-phenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methylphenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-ditert-butyl-4-hydroxyphenyl)-propan, 2,2-Bis-(5-tert-butyl-4-hydroxy-2-methylphenyl)-4-ndodecylmercapto-butan, 1,1,5,5-Tetra-(5-tert-butyl-4-hydroxy-2-methylphenyl)-pentan.
   1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, isooctyl-3,5-di-tert-butyl-4-hydroxybenzylmercaptoacetat.
   1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-ditert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.12. Ester der β-(3,5-Di-tert-butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol; Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der 3.5 -Di -tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Amide der β-(3,5-Di-tert-butyl-4-hydroxyphenyl)-propionsäure, wie z.B. N,N'-Bis-(3,5-di-tert-butyl-4-hydroxyphenylproplonyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.
2. UV-Absorber und Lichtschutzmittel
   2.1. 2-(2'-Hydroxyphenyl)-benztriazole, wie z.B. 2-(2'-Hydroxy-5'-methylphenyl)-benztriazol, 2-(3',5'-Di-tert-butyl-2'-hydroxyphenyl)-benztriazol. 2-(5'-tert-Butyl-2'-hydroxyphenyl)-benztriazol, 2-(2'-Hydroxy-5'-(1,1,3,3-tetramethylbutyl)phenyl)-benztriazol, 2-(3',5'-Di-tert-butyl-2'hydroxyphenyl)-5-chlor-benztriazol, 2-(3'-tert.Butyl- 2'-hydroxy-5'-methylphenyl)-5-chtorbenztriazol, 2-(3'-sec-Butyl-5'-tert-butyl-2'-hydroxyphenyl)-benztriazol, 2-(2'-Hydroxy-4'octoxyphenyl)-benztriazol, 2-(3',5'-Di-tert-amyl-2'-hydroxyphenyl)-benztriazol, 2-(3',5'-Bis--(α,α-dimethylbenzyl)-2'-hydroxyphenyl)-benztriazol, Mischung aus 2-(3'-tert-Butyl-2'-hydroxy-5'-(2-octyloxycarbonylethyl)phenyl)-5-chlor-benztriazol, 2-(3'-tert-Butyl-5'-[2-(2-ethylhexyloxy)-carbonylethyl]-2'-hydroxyphenyl)-5-chlor-benztriazol, 2-(3'-tert-Butyl-2'hydroxy-5'-(2-methoxycarbonylethyl)phenyl)-5-chlor-benztriazol, 2-(3'-tert-Butyl-2'-hydroxy--5'-(2-methoxycarbonylethyl)phenyl)-benztriazol, 2-(3'-tert-Butyl-2'-hydroxy-5'-(2-octyloxycarbonylethyl)phenyl)-benztriazol, 2-(3'-tert-Butyl-5'-[2-(2ethylhexyloxy)carbonylethyl]-2'-hydroxyphenyl)-benztriazol, 2-(3'-Dodecyl-2'-hydroxy-5'-methylphenyl)-benztriazol, und 2-(3'-tert-Butyl-2'-hydroxy-5'-(2-isooctyloxycarbonylethyl)phenyl-benztriazol, 2,2'--Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; Verbindungen der Formel

      [R-CH₂CH₂COO(CH₂)₃]₂

      mit R = 3'-tert-Butyl-4'-hydroxy-5'-2H-benztriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. a-Cyan-β,β-diphenylacrylsäure-ethylester bzw. -isooctylester, α-Carbomethoxy-zimtsäuremethylester, a-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl--7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-nbutylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Weitere Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkaliund Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863 oder US-A-4 338 244 beschrieben.

Von besonderer Bedeutung sind stabilisierte Polymere, welche einen zusätzlichen Gehalt an einem Lichtschutzmittel aus der Klasse der sterisch gehinderten Amine oder/und aus der Klasse der 2-(2'-Hydroxyphenyl)-benztriazole aufweisen. Unter den sterisch gehinderten Aminen sind insbesondere solche Verbindungen zu verstehen, die im Molekül eine oder mehrere Gruppen der Formel 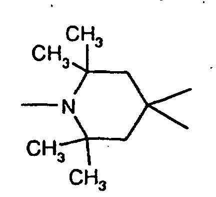 enthalten, wobei diese Verbindungen monomer, oligomer oder polymer sein können. Beispiele für solche Verbindungen sind obigem Punkt 2.6. der Liste von möglichen zusätzlichen Stabilisatoren zu entnehmen.

Der Zusatz der Verbindungen der Formel (1) sowie gegebenenfalls weiterer Zusatzstoffe zu den Polymeren kann vor oder während der formgebenden Verarbeitung der Polymeren erfolgen, beispielsweise durch Mischen in Pulverform oder durch Zusatz zur Schmelze oder Lösung des Polymeren bzw. zu einer geeigneten Lackformulierung, die einen polymeren Binder enthält.

Die Erfindung betrifft daher auch die durch Zusatz mindestens einer Verbindung der Formel (1) stabilisierten Polymeren, die gegebenenfalls noch andere Zusatzstoffe enthalten können. Die so stabilisierten Polymeren können in verschiedener Form verwendet werden, wie z.B. als Fasern, Folien, Faserbändchen, Profile, Hohlkörper, Platten, Doppelstegplatten oder als Bindemittel für Lacke, Anstriche, Klebstoffe und Kitte. Von besonderem Interesse ist ihre Verwendung in Lacken.

Die neuen UV-Absorber eignen sich ausserdem als Lichtschutzmittel in kosmetischen Präparaten, z.B. in Haut- oder Haarpflegemitteln.

Einen weiteren Erfindungsgegenstand bildet daher ein kosmetisches Präparat enthaltend mindestens eine Verbindung der allgemeinen Formel (1) sowie kosmetisch verträgliche Träger- oder Hilfsstoffe.

Für die kosmetische Verwendung haben die erfindungsgemässen Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Die kosmetischen Zubereitungen können neben den erfindungsgemässen UV-Absorbern auch noch einen oder mehrere weitere UV-Absorber, wie z.B. Oxanilide, Triazole, Vinylgruppen enthaltende Amide oder Zimtsäureamide enthalten.

Als Oxanilide kommen z.B. Verbindungen der Formel 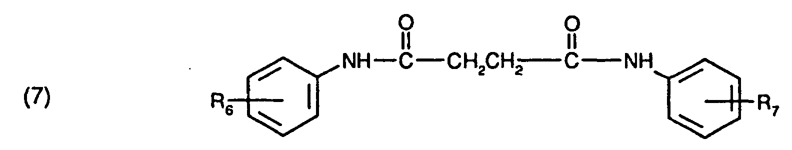 worin
R₆ und R₇, unabhängig voneinander, C₁-C₁₈-Alkyl oder C₁-C₁₈-Alkoxy bedeuten, in Betracht.

Bevorzugte Triazolverbindungen entsprechen der Formel 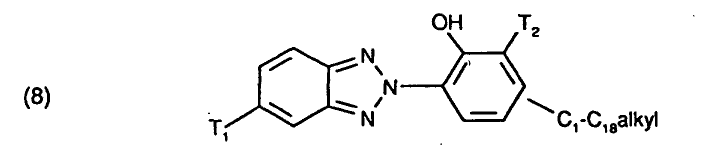 worin
- T₁: C₁-C₁₈-Alkyl, oder vorzugsweise Wasserstoff; und
- T₂: gegebenenfalls durch eine Phenylgruppe substituiertes C₁-C₁₈-Alkyl bedeutet.

Eine weitere Klasse von Triazol-Verbindungen entspricht der Formel 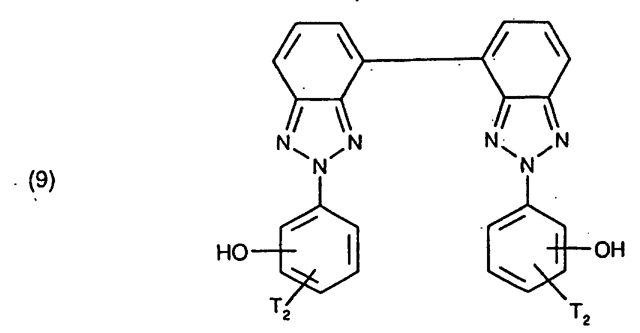 worin
T₂ die in Formel (8) angegebene Bedeutung hat.

Bevorzugte Vinylgruppen enthaltende Amide entsprechen der Formel

(10) R₉-(Y)ₙ-C(=O)-C(R₁₀)=C(R₁₁)-N(R₁₂)(R₁₃),

worin
R₉ C₁-C₁₈-Alkyl, vorzugweise C₁-C₅-Alkyl oder Phenyl, wobei Phenyl durch zwei oder drei Substituenten ausgewählt aus Hydroxy, C₁-C₁₈-Alkyl oder C₁-C₁₈-Alkoxy oder einer -C(=O)-OR₈-Gruppe, worin
- R₈: C₁-C₁₈-Alkyl ist, substituiert ist;
- R₁₀ , R₁₁, R₁₂ und R₁₃,: unabhängig voneinander, Waasserstoff oder C₁-C₁₈-Alkyl;
- Y: N oder O; und
- n: 0 oder 1 bedeuten.

Bevorzugte Zimtsäurederivate entsprechen der Formel 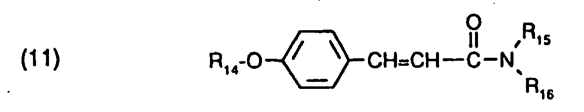 worin
- R₁₄: Hydroxy oder C₁-C₄-Alkoxy, vorzugsweise Methoxy oder Ethoxy;
- R₁₅: Wasserstoff oder C₁-C₄-Alkyl, vorzugsweise Methyl oder Ethyl;
- R₁₆: -(CONH)ₙ-phenyl,
- n: 0 oder 1 bedeuten und
der Phenylring gegebenenfalls durch einen, zwei oder drei Substituenten, ausgewählt aus OH, C₁-C₁₈-Alkyl oder C₁-C₁₈-Alkoxy oder einer -C(=O)-OR₈-Gruppe, wobei R₈ die oben angegebene Bedeutung hat, substituiert sein kann.

Die neben den erfindungsgemässen UV-Absorbern eingesetzten zusätzlichen UV-Absorbern sind z.B. aus Cosmetics & Toiletries (107), 50ff (1992) bekannt.

Die erfindungsgemässe kosmetische Zusammensetzung enthält 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung der kosmetischen Zusammensetzung kann durch physikalisches Mischen des oder der UV-Absorber mit dem Hilfsstoff durch gewöhnliche Methoden, wie z.B. durch einfaches Zusammenrühren der zwei Materialien erfolgen.

Die erfindungsgemässe kosmetische Zubereitung kann als Wasser-in-Öl- oder Öl-in-Wassr-Emulsion, als Öl-in-Öl-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfsstoff vorzugsweise 5 bis 50 % einer Ölphase, 5 bis 20 % eines Emulgators und 30 bis 90% Wasser. Die Ölphase kann dabei igendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Für die erfindungsgemässen kosmetischen Formulierungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-Öl; oder ein Silikonöl-Emulgator wie z.B. Silikonpolyol; eine gegebenenfalls ethoxylierte Fettsäureseife; ein ethoxyxlierter Fettalkohol; ein gegebenenfalls ethoxylierter Sorbitanester; eine ethoxyoierte Fettsäure; oder ein ethoxyliertes Glycerid.

Die kosmetische Forulierung kann auch weitere Komponenten wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe.

Die erfindungsgemässen kosmetischen Formulierungen zeichnen sich durch exzellenten Schutz der menschlichen Haut gegen den schädigenden Einfluss von Sonnenlicht aus bei gleichzeitiger sicherer Bräunung der Haut. Darüber hinaus sind die erfindungsgemässen kosmetischen Zubereitungen beim Auftragen auf der Haut wasserfest.

In den folgenden Beispielen beziehen sich die Prozentsätze auf das Gewicht. Die Mengen beziehen sich bei den Farbstoffen und bei den Triazinverbindungen auf die Reinsubstanz.

### Beispiel 12: 2-{4,6-Bis-[4-(2-ethylhexyloxy)-phenyl]-s-triazin-2-yl}-5-(2-ethylhexyloxy)-phenol

a) In einem 1I Sulfierkolben mit Rührer, Kühler, Tropftrichter und Innenthermometer werden 61,4 g (0,5 Mol) 4-Hydroxybenzonitril In 500 ml Methylcellosolve vorgelegt. Nach Erwärmen des Ansatzes auf 80°C lässt man unter kräftigem Rühren langsam 73,3 g 30%-ige NaOH (0,55 Mol) einlaufen. Es wird 15 Minuten nachgerührt, bevor man während 30 Minuten 116,8 g (0,575 Mol) 3-Brommethylheptan zutropft. Die Reaktion wird für ca. 12 Stunden bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 500 ml Toluol auf. Es wird dreimal mit Wasser ausgeschüttelt, über Natriumsulfat getrocknet und zur Trockne eingedampft. Nach Hochvakuumdestillation über eine 10cm Vigreux-Kolonne (127-132°C, 0,15 mm) erhält man 97,5 g (84% d. Th.) 4-(2-Ethylhexyloxy)-benzonitril als farbiges Öl.
b) In einem 1,51 Planschliffreaktor, ausgestattet mit Rührer, Kühler, Innenthermometer und Gaseinleitungsrohr, werden 208,7 g (0,9 Mol) 4-(2-Ethylhexyloxy)benzonitril sowie 39,8g (1,22 Mol) Methanol in 400 ml Dichlorethan vorgelegt. Innerhalb von 5 Stunden leitet man unter starkem Rühren und Eiskühlung (0-1°C) 85,4 g (2,37 Mol) Chlorwasserstoffgas ein. Nach 24-stündigem Rühren bei Raumtemperatur zeigt das Dünnschichtchromatogramm eine quantitative Umsetzung zum lmidoester. Man zieht das Lösungsmittel im Vakuum ab und lässt den gelben, viskosen Rückstand unter Eiskühlung (0-10°C) innerhalb von 30 Minuten in eine gut gerührte Lösung von 34 g (2,0 Mol) Ammoniak in 800 ml Methanol einlaufen. Man rührt 1 Stunde bei Raumtemperatur, dann weitere 90 Minuten bei 50-60°C nach. Der Ansatz wird im Vakuum zur Trockne eingedampft, der schmierige Rückstand dann in 800 ml warmem Toluol/Ethanol (8/2) verrührt und über Kieselgel filtriert. Dadurch wird ein Grossteil des anfallenden Ammoniumchlorids abgetrennt. Nach Einengen des Fitrats wird diese Reinigung noch zweimal wiederholt. Man erhält 205 g (80% d.Th.) Amidiniumsalz (Fp. 172-173°C), welches noch geringe Mengen Ammoniumchlorid enthält.
c) In einem 2,51 Sulfierkolben mit Rührer, Kühler, Innenthermometer und Tropftrichter und pH-Elektrode werden 113,9 g (0,4 Mol) des in b) erhaltenen Amidiniumsalzes in einem Gemisch von 1000 ml Wasser (dest.) und 100 ml Aceton suspendiert. Bei 15-20°C werden 106,7 g 30%-ige Natronlauge (0,8 Mol) während 30 Minuten langsam zugegeben. Anschliessend tropft man 45,6 g (0,42 Mol) Chlorameisensäureethylester innerhalb einer Stunde zu (Innentemperatur: 15-20°C). Im Laufe der Umsetzung sinkt der pH-Wert von 13 (Anfangswert) auf 7,0-7,5 ab und man erhält eine körnige Suspension. Nach Zugabe von 500 ml 1,2-Dichlorbenzol wird unter Rühren auf 80°C erwärmt. Man trennt die organische Phase im Scheidetrichter ab, überführt sie in einen 1,51 Sulfierkolben (ausgestattet mit einem Liebigkühler) und erhitzt unter leichtem Vakuum (ca. 800 mbar) auf 145-175°C (Innentemperatur). Das bei der Ringschlusskondensation entstehende Urethan wird abdestilliert (Dauer ca. 90 Minuten). Man lässt die braune Reaktionsmasse bei 60°C in 600 ml Isopropanol einlaufen, saugt den Niederschlag in der Kälte (5°C) ab und wäscht mit Isopropanol, Wasser und Methanol. Anschliessend wird im Vakuum getrocknet (100°C). Das Produkt 4,6-Bis[4-(2-ethyl-hexyloxy)-phenyl]-s-triazin-2-ol zeigt eine blaue Fluoreszenz und ist dünnschichtchromatographisch einheitlich. Die Ausbeute beträgt 57 g (56 % d. Th.; Fp. 168-170°C).
d) In einem 1,5I Sulfierkolben mit Rührer, Kühler, Innenthermometer, Tropftrichter und Gasableitung werden 55,6 g (0,11 Mol) 4,6-Bis[4-(2-ethyl-hexyloxy)-phenyl]-s-triazin-2-ol in 300 ml Xylol, versetzt mit 1 ml Dimethylformamid, vorgelegt. Bei 75-80°C Innentemperatur tropft man unter starkem Rühren während 15 Minuten 17,0 g (0,14 Mol) Thionylchlorid zu. Nach Abklingen der Gasentwicklung wird die Temperatur auf 100°C erhöht. Nach 2 Stunden ist die Umsetzung beendet (Prüfung durch Dünnschichtchromatographie). Überschüssiges Thionylchorid wird unter leichtem Vakuum aus dem Reaktionsgefäss abdestilliert, das Zwischenprodukt 2-Chloro-4,6-bis-[4-(2-ethyl-hexyloxy)-phenyl]-s-triazin wird direkt weiter umgesetzt. Man trägt bei 50°C 16,2 g (0,12 Mol) trockenes, sublimiertes Aluminiumchlorid ein (ca. 1 Minute), wodurch die Temperatur auf 65°C ansteigt. Die anfangs klare, gelbe Lösung wird zu einer roten, dann olivefarbenen Suspension. Bei 50-55°C werden portionsweise 13,3 g (0,12 Mol) Resorcin zugegeben (ca. 10 Minuten), anschliessend wird auf 85°C erwärmt. Nach 3 Stunden ist das Dünnschichtchromatogramm frei von Edukt. Man kühlt auf 70°C ab und hydrolisiert den Aluminiumkomplex durch langsames Zutropfen von 300 ml 5%-iger Salzsäure, wobei die Temperatur von 80°C nicht überschritten werden sollte. Das Lösungsmittel (Xylol) wird durch Wasserdampfdestillation entfernt, der schmierige Rückstand in 500 ml heissem Toluol verrührt und über Kieselgel filtriert. Das Filtrat wird

### Beispiel 12: 2-{4,6-Bis-[4-(2-ethylhexyloxy)-phenyl]-s-triazin-2-yl}-5-(2-ethylhexyloxy)-phenol

a) In einem 1I Sulfierkolben mit Rührer, Kühler, Tropftrichter und Innenthermometer werden 61,4 g (0,5 Mol) 4-Hydroxybenzonitril in 500 ml Methylcellosolve vorgelegt. Nach Erwärmen des Ansatzes auf 80°C lässt man unter kräftigem Rühren langsam 73,3 g 30%-ige NaOH (0,55 Mol) einlaufen. Es wird 15 Minuten nachgerührt, bevor man während 30 Minuten 116,8 g (0,575 Mol) 3-Brommethylheptan zutropft. Die Reaktion wird für ca. 12 Stunden bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 500 ml Toluol auf. Es wird dreimal mit Wasser ausgeschüttelt, über Natriumsulfat getrocknet und zur Trockne eingedampft. Nach Hochvakuumdestillation über eine 10cm Vigreux-Kolonne (127-132°C, 0,15 mm) erhält man 97,5 g (84% d. Th.) 4-(2-Ethylhexyloxy)-benzonitril als farbiges Öl.
b) In einem 1,51 Planschliffreaktor, ausgestattet mit Rührer, Kühler, Innenthermometer und Gaseinleitungsrohr, werden 208,7 g (0,9 Mol) 4-(2-Ethylhexyloxy)benzonitril sowie 39,8g (1,22 Mol) Methanol in 400 ml Dichlorethan vorgelegt. Innerhalb von 5 Stunden leitet man unter starkem Rühren und Eiskühlung (0-1°C) 85,4 g (2,37 Mol) Chlorwasserstoffgas ein. Nach 24-stündigem Rühren bei Raumtemperatur zeigt das Dünnschichtchromatogramm eine quantitative Umsetzung zum Imidoester. Man zieht das Lösungsmittel im Vakuum ab und lässt den gelben, viskosen Rückstand unter Eiskühlung (0-10°C) innerhalb von 30 Minuten in eine gut gerührte Lösung von 34 g (2,0 Mol) Ammoniak in 800 ml Methanol einlaufen. Man rührt 1 Stunde bei Raumtemperatur, dann weitere 90 Minuten bei 50-60°C nach. Der Ansatz wird im Vakuum zur Trockne eingedampft, der schmierige Rückstand dann in 800 ml warmem Toluol/Ethanol (8/2) verrührt und über Kieselgel filtriert. Dadurch wird ein Grossteil des anfallenden Ammoniumchlorids abgetrennt. Nach Einengen des Filtrats wird diese Reinigung noch zweimal wiederholt. Man erhält 205 g (80% d.Th.) Amidiniumsalz (Fp. 172-173°C), welches noch geringe Mengen Ammoniumchlorid enthält.
c) In einem 2,51 Sulfierkolben mit Rührer, Kühler, Innenthermometer und Tropftrichter und pH-Elektrode werden 113,9 g (0,4 Mol) des in b) erhaltenen Amidiniumsalzes in einem Gemisch von 1000 ml Wasser (dest.) und 100 ml Aceton suspendiert Bei 15-20°C werden 106,7 g 30%-ige Natronlauge (0,8 Mol) während 30 Minuten langsam zugegeben. Anschliessend tropft man 45,6 g (0,42 Mol) Chlorameisensäureethylester innerhalb einer Stunde zu (Innentemperatur: 15-20°C). Im Laufe der Umsetzung sinkt der pH-Wert von 13 (Anfangswert) auf 7,0-7,5 ab und man erhält eine körnige Suspension. Nach Zugabe von 500 ml 1,2-Dichlorbenzol wird unter Rühren auf 80°C erwärmt. Man trennt die organische Phase im Scheidetrichter ab, überführt sie in einen 1,51 Sulfierkolben (ausgestattet mit einem Liebigkühler) und erhitzt unter leichtem Vakuum (ca. 800 mbar) auf 145-175°C (Innentemperatur). Das bei der Ringschlusskondensation entstehende Urethan wird abdestilliert (Dauer ca. 90 Minuten). Man lässt die braune Reaktionsmasse bei 60°C in 600 ml Isopropanol einlaufen, saugt den Niederschlag in der Kälte (5°C) ab und wäscht mit Isopropanol, Wasser und Methanol. Anschliessend wird im Vakuum getrocknet (100°C). Das Produkt 4,6-Bis[4-(2-ethyl-hexyloxy)-phenyl]-s-triazin-2-ol zeigt eine blaue Fluoreszenz und ist dünnschichtchromatographisch einheitlich. Die Ausbeute beträgt 57 g (56 % d. Th.; Fp. 168-170°C).
d) In einem 1,51 Sulfierkolben mit Rührer, Kühler, Innenthermometer, Tropftrichter und Gasableitung werden 55,6 g (0,11 Mol) 4,6-Bis[4-(2-ethyl-hexyloxy)-phenyl]-s-triazin-2-ol in 300 ml Xylol, versetzt mit 1 ml Dimethylformamid, vorgelegt. Bei 75-80°C Innentemperatur tropft man unter starkem Rühren während 15 Minuten 17,0 g (0,14 Mol) Thionylchlorid zu. Nach Abklingen der Gasentwicklung wird die Temperatur auf 100°C erhöht. Nach 2 Stunden ist die Umsetzung beendet (Prüfung durch Dünnschichtchromatographie). Überschüssiges Thionylchorid wird unter leichtem Vakuum aus dem Reaktionsgefäss abdestilliert, das Zwischenprodukt 2-Chloro-4,6-bis-[4-(2-ethyl-hexyloxy)-phenyl]-s-triazin wird direkt weiter umgesetzt. Man trägt bei 50°C 16,2 g (0,12 Mol) trockenes, sublimiertes Aluminiumchlorid ein (ca. 1 Minute), wodurch die Temperatur auf 65°C ansteigt. Die anfangs klare, gelbe Lösung wird zu einer roten, dann olivefarbenen Suspension. Bei 50-55°C werden portionsweise 13,3 g (0,12 Mol) Resorcin zugegeben (ca. 10 Minuten), anschliessend wird auf 85°C erwärmt. Nach 3 Stunden ist das Dünnschichtchromatogramm frei von Edukt. Man kühlt auf 70°C ab und hydrolisiert den Aluminiumkomplex durch langsames Zutropfen von 300 ml 5%-iger Salzsäure, wobei die Temperatur von 80°C nicht überschritten werden sollte. Das Lösungsmittel (Xylol) wird durch Wasserdampfdestillation entfemt, der schmierige Rückstand in 500 ml heissem Toluol verrührt und über Kieselgel filtriert. Das Filtrat wird mit Aktivkohle digeriert und nochmals filtriert. Man trocknet mit Natriumsulfat und destilliert das Lösungsmittel ab. Der braune, viskose Rückstand (69 g) wird zur weiteren Reinigung in 150 ml Toluol/Essigester (95/5) gelöst und einer Säulenchromatographie unterworfen (6 cm X 60 cm Kieselgel 60). Man erhält 33,6 g (51%d.Th.) 4-{4,6-Bis-[4-(2-ethyl-hexyloxy)-phenyl]-s-triazin-2-yl}-1,3-dihydroxybenzol als gelbes viskoses Öl.
e) 83,7 g (0,14 Mol) 4-{4,6-Bis-[4-(2-ethyl-hexyloxy)-phenyl]-s-triazin-2-yl}-1,3- dihydroxybenzol werden zusammen mit 500 ml Methylcellosolve in einem 11 Sulfierkolben mit Rührer, Kühler, Innenthermometer und Tropftrichter bei 80°C vorgelegt. Man gibt 18,1 g einer 30%igen Natronlauge (0,16 Mol) zu, lässt 15 Minuten nachrühren und tropft dann während 30 Minuten eine Lösung von 31,4 g (0,16 Mol) 3-Brommethylheptan und 30 ml Methylcellosolve zu. Nach 24 Stunden Rühren bei 110°C ist die Alkylierung beendet (Dünnschichtchromatogramm). Es wird unter Vakuum zur Trockne eingedampft, der Rückstand in 500 ml Toluol gelöst, filtriert und mit Wasser ausgeschüttelt. Nach Trocknung über Natriumsulfat und Abdestillieren des Lösungsmittels erhält man 100,9 g eines rotbraunen Öls. Das Rohprodukt wird in 200 ml Toluol/Essigester (97,5/2,5) gelöst und zur Reinigung über Kieselgel (10 cmx40 cm) chromatographiert. Man erhält 79,4 g (80% d.Th.) der Verbindung der Formel 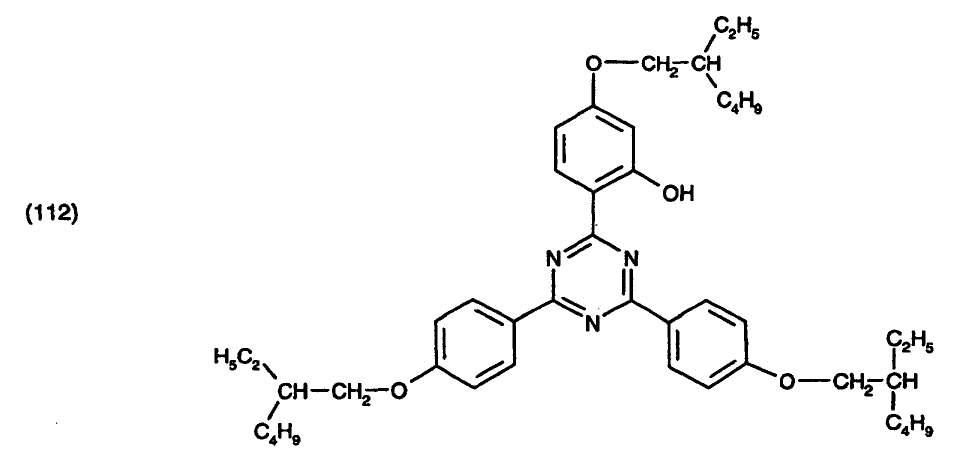 als honigartiges, rotbraunes Harz.

### Beispiel 14: 2-{4,6-Bis-[4-(2-ethylhexyloxy)-phenyl]-s-triazin-2-yl}-5-(2-ethylhexyloxy)-phenol

a) In einem 1I Sulfierkolben mit Rührer, Kühler, Tropftrichter und Innenthermometer werden 86,5 g (0,5 Mol) 4-Bromphenol in 500 ml Methylcellosolve vorgelegt. Nach Erwärmen auf 60°C lässt man langsam 70,0 g 30%-ige NaOH (0,53 Mol) einlaufen. Es wird 15 Minuten nachgerührt, bevor man während 45 Minuten 116,8 g (0,575 Mol) 3-Brommethylheptan zutropft. Die Reaktion wird über Nacht bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 600 ml Toluol auf. Es wird dreimal mit Wasser ausgeschüttelt, über Natriumsulfat getrockent und zur Trockne eingedampft. Man erhält 119,3 g (84% d.Th.) 4-(2-Ethylhexyloxy)-brombenzol als leichtes gelbes Öl.
b) In einem 100 ml Sulfierkolben, ausgerüstet mit Rührer, Kühler, Trockenrohr, Tropftrichter und Innenthermometer werden unter Schutzgas (trockener Stickstoff) 3,65 g (0,15 Mol) Magnesiumspäne vorgelegt und mit einigen Kristallen Jod angeätzt. Man fügt 150ml was serfreies Tetrahydrofuran hinzu und tropft innerhalb von 45 Minuten eine Lösung von 42,8 g (0,15 Mol) 4-(2-Ethylhexyloxy)-brombenzol in 30 ml Tetrahydrofuran zu (Raumtemperatur). Nach leichtem Erwärmen auf dem Wasserbad (40°C) springt die Grignard-Reaktion an Trübung, Exothermie). Man rührt eine Stunde bei 40°C, dann unter Rückfluss (66°C), bis das Magnesium nahezu vollständig gelöst ist (ca. 30 Minuten). Nach Abkühlung auf Raumtemperatur wird die Grignard-Lösung bei 0-20°C innerhalb von 60 Minuten zu einer Lösung von 9,2 g (0,05 Mol) Cyanurchlorid in 40 ml Tetrahydrofuran zugetropft (350 ml Sulfierkolben, Rührer, Kühler, Trockenrohr, Tropftrichter, Innenthermometer, Schutzgas). Man rührt über Nacht unter Rückfluss (66°C) und dampft anschliessend zur Trockne ein. Der Rückstand wird in 100 ml eiskalter 2N Salzsäure verrührt und dann mit 200 ml Toluol extrahiert. Die organische Phase wird zweimal mit 10%-iger Sole geschüttelt, über Natriumsulfat getrocknet und eingedampft. Das Rohprodukt (40,3 g, rotes Öl) ist noch stark verunreinigt (Dünnschichtchromatogramm). Zur Reinigung wird in 80 ml Toluol/Hexan (1/1) gelöst und über Kieselgel (5 cm x 45 cm) chromatographiert. Man isoliert 18,2 g (69,5%) 2-Chloro-4,6-bis-[4-(2-ethyl-hexyloxy)-phenyl]-s-triazin als gelbes Harz.
c) Die Friedel-Crafts-Acylierung erfolgt wie in Beispiel 12 d) beschrieben. Man erhält das Produkt 4-{4,6-Bis-[4-(2-ethylhexyloxy)-phenyl]-s-triazin-2-yl}-1,3- dihydroxybenzol.
d) Die Alkylierung erfolgt wie in Beispiel 12 e) beschrieben.

Das Endprodukt ist die Verbindung der Formel (112).

### Beispiel 15 bis 17:

a) In einem 1l Sulfierkolben mit Rührer, Kühler, Troaftrichter und Innenthermometer werden 61.4 g (0,5 Mol) 4-Hydroxybenzonitril in 500 ml Methylcellosolve vorgelegt. Nach Erwärmen des Ansatzes auf 80°C lässt man unter kräftigem Rühren langsam 73.3 g 30%-ige NaOH (0,55 Mol) einlaufen. Es wird 15 Minuten nachgeführt, bevor man während 30 Minuten 116,8 g (0,575 Mol) 3-Brommethylheptan zutronft. Die Reaktion wird für ca. 12 Stunden bei 100°C fortgesetzt. Im Dünnschichtchromatogramm erkennt man nahezu quantitativen Umsatz. Man entfernt Lösungsmittel und überschüssiges Bromid im Vakuum und nimmt den Rückstand (Öl) in 500 ml Toluol auf. Es wird dreimal mit Wasser ausgeschüttelt, über Natriumsulfat getrocknet und zur Trockne eingedampft. Nach Hochvakuumdestillation über eine 10cm Vigreux-Kolonne (127-132°C, 0,15 mm) erhält man 97,5 g (84% d. Th.) 4-(2-Ethylhexyloxy)-benzonitril als farbiges Öl.
b) In einem 1,51 Planschliffreaktor, ausgestattet mit Rührer, Kühler, Innenthermometer und Gaseinleitungsrohr, werden 208,7 g (0,9 Mol) 4-(2-Ethylhexyloxy)benzonitril sowie 39,8g (1,22 Mol) Methanol in 400 ml Dichlorethan vorgelegt. Innerhalb von 5 Stunden leitet man unter starkem Rühren und Eiskühlung (0-1°C) 85,4 g (2,37 Mol) Chlorwasserstoffgas ein. Nach 24-stündigem Rühren bei Raumtemperatur zeigt das Dünnschichtchromatogramm eine quantitative Umsetzung zum Imidoester. Man zieht das Lösungsmittel im Vakuum ab und lässt den gelben, viskosen Rückstand unter Eiskühlung (0-10°C) innerhalb von 30 Minuten in eine gut gerührte Lösung von 34 g (2,0 Mol) Ammoniak in 800 ml Methanol einlaufen. Man rührt 1 Stunde bei Raumtemperatur, dann weitere 90 Minuten bei 50-60°C nach. Der Ansatz wird im Vakuum zur Trockne eingedampft, der schmierige Rückstand dann in 800 ml warmem Toluol/Ethanol (8/2) verrührt und über Kieselgel filtriert. Dadurch wird ein Grossteil des anfallenden Ammoniumchlorids abgetrennt. Nach Einengen des Filtrats wird diese Reinigung noch zweimal wiederholt. Man erhält 205 g (80% d.Th.) Amidiniumsalz (Fp. 172-173°C), welches noch geringe Mengen Ammoniumchlorid enthält.
c) In einem 2,51 Sulfierkolben mit Rührer, Kühler, Innenthermometer und Tropftrichter und pH-Elektrode werden 113,9 g (0,4 Mol) des in b) erhaltenen Amidiniumsalzes in einem Gemisch von 1000 ml Wasser (dest.) und 100 ml Aceton suspendiert. Bei 15-20°C werden 106,7 g 30%-ige Natronlauge (0,8 Mol) während 30 Minuten langsam zugegeben. Anschliessend tropft man 45,6 g (0,42 Mol) Chlorameisensäureethylester innerhalb einer Stunde zu (Innentemperatur: 15-20°C). Im Laufe der Umsetzung sinkt der pH-Wert von 13 (Anfangswert) auf 7,0-7,5 ab und man erhält eine körnige Suspension. Nach Zugabe von 500 ml 1,2-Dichlorbenzol wird unter Rühren auf 80°C erwärmt. Man trennt die organische Phase im Scheidetrichter ab, überführt sie in einen 1,51 Sulfierkolben (ausgestattet mit einem Liebigkühler) und erhitzt unter leichtem Vakuum (ca. 800 mbar) auf 145-175°C (Innentemperatur). Das bei der Ringschlusskondensation entstehende Urethan wird abdestilliert (Dauer ca. 90 Minuten). Man lässt die braune Reaktionsmasse bei 60°C in 600 ml Isopropanol einlaufen, saugt den Niederschlag in der Kälte (5°C) ab und wäscht mit Isopropanol, Wasser und Methanol. Anschliessend wird im Vakuum getrocknet (100°C). Das Produkt 4,6-Bis[4-(2-ethyl-hexyloxy)-phenyl]-s-triazin-2-ol zeigt eine blaue Fluoreszenz und ist dünnschichtchromatographisch einheitlich. Die Ausbeute beträgt 57 g (56 % d. Th.; Fp. 168-170°C).
d) In einem 1,5I Sulfierkolben mit Rührer, Kühler, Innenthermometer, Tropftrichter und Gasableitung werden 55.6 g (0.11 Mol) 4,6-Bis[4-(2-ethyl-hexyloxy)-phenyl]-s-triazin-2-ol in 300 ml Xylol, versetzt mit 1 ml Dimethylformamid, vorgelegt. Bei 75-80°C Innentemperatur tropft man unter starkem Rühren während 15 Minuten 17.0 g (0.14 Mol) Thionylchlorid zu. Nach Abklingen der Gasentwicklung wird die Temperatur auf 100°C erhöht. Nach 2 Stunden ist die Umsetzung beendet (Prüfung durch Dünnschichtchromatographie). Überschüssiges Thionylchorid wird unter leichtem Vakuum aus dem Reaktionsgefäss abdestilliert, das Zwischenprodukt 2-Chloro-4.6-bis-[4-(2-ethyl-hexyloxy)-phenyl]-s-triazin wird direkt weiter umgesetzt. Man trägt bei 50°C 16,2 g (0.12 Mol) trockenes, sublimiertes Aluminiumchlorid ein (ca. 1 Minute), wodurch die Temperatur auf 65°C ansteigt. Die anfangs klare, gelbe Lösung wird zu einer roten, dann olivefarbenen Suspension. Bei 50-55°C werden portionsweise 0,12 Mol Phenol zugegeben (ca. 10 Minuten), anschliessend wird auf 85°C erwärmt. Nach 3 Stunden ist das Dünnschichtchromatogramm frei von Edukt. Man kühlt auf 70°C ab und hydrolisiert den Aluminiumkomplex durch langsames Zutropfen von 300 ml 5%iger Salzsäure, wobei die Temperatur von 80°C nicht überschritten werden sollte. Das Lösungsmittel (Xylol) wird durch Wasserdampfdestillation entfernt, der schmierige Rückstand in 500 ml heissem Toluol verrührt und über Kieselgel filtriert. Das Filtrat wird mit Aktivkohle digeriert und nochmals filtriert. Man trocknet mit Natriumsulfat und destilliert das Lösungsmittel ab. Der braune, viskose Rückstand (69 g) wird zur weiteren Reinigung in 150 ml Toluol/Essigester (95/5) gelöst und einer Säulenchromatographie unterworfen (6 cm X 60 cm Kieselgel 60). Man erhält 33.6 a (51%d.Th.) 4-{4,6-Bis-[4-(2-ethyl-hexyloxy)-phenyl]-striazin-2-yl}-1-hydroxybenzol als gelbes viskoses Öl.

Nach den in den Beispielen 12 bis 14 beschriebenen Verfahren sind auch die folgenden Verbindungen erhältlich (Tabelle 2):

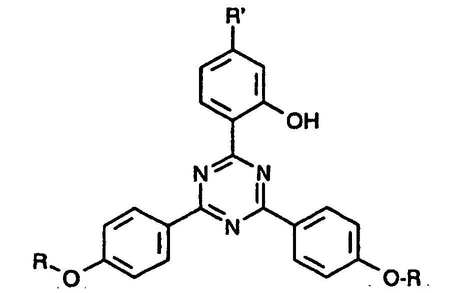

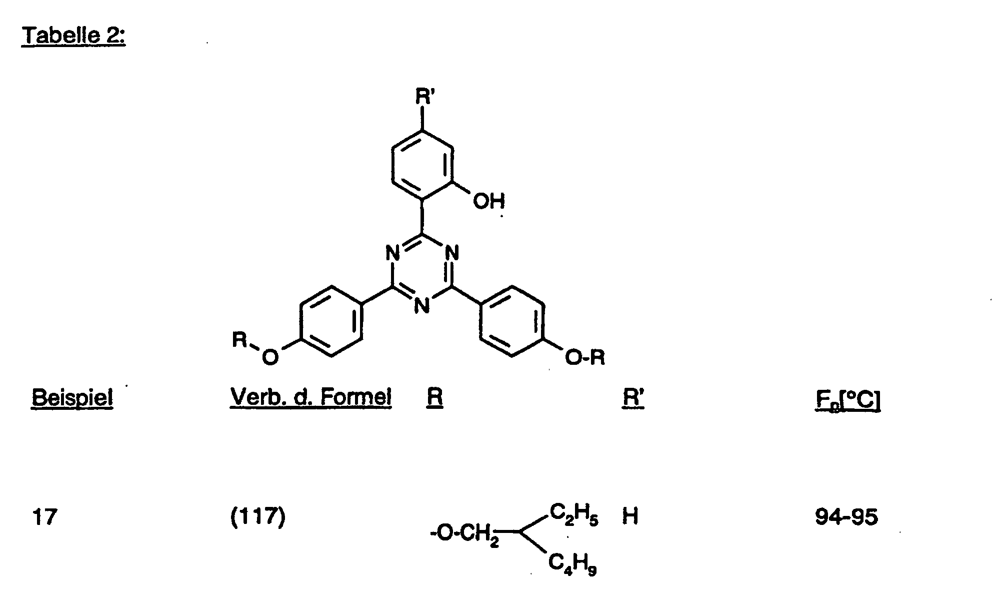

### Beispiel 18: Weitere Herstellungvariante der Verbindung der Formel (117):

a. Herstellung von 2-[4,6-Bis-(4-hydroxyphenyl)-s-triazin-2-yl]-phenol: In einem 750 ml Sulfierkolben mit Rührer, Kühler, Innenthermometer und Tropftrichter werden 9.2g (0,4 mol) Natrium in 200ml Ethanol abs. gelöst und anschließend bei 10-15°C mit 72,7g (0,4 mol; 95% rein) 4-Hydroxy-benzamidinium-chlorid (Herstellung analog Beispiel 12b)) versetzt. Man rührt 15 Minuten bei derselben Temperatur nach (weiße Kochsalz-Suspension). Nun werden 85,7 g (0,4 mol) Salicylsäure-phenylester, gelöst in 150 ml Ethanol abs., langsam zugegeben. Der Ansatz wird auf Rückflusstemperatur (78°C) erhitzt, was unter Gasentwicklung zur Bildung einer gelben Suspension führt. Man hält über Nacht (18 Stunden) am Rückfluss und dampft im Vakuum zur Trockene ein. Der Rückstand wird in 250 ml Wasser verrührt (alkalische Suspension), mit Salzsäure schwach sauer gestellt und wiederholt mit Wasser verrührt. Man suspendiert die halbfeste Masse in 50 ml Isopropanol, saugt ab und wäscht mit Isopropanol. Die Trocknung erfolgt bei 100°C im Vakuum. Das Rohprodukt, welches dünnschichtchromatographisch noch leicht verunreinigt ist, kann durch Umkristallisation aus Dimethylformamid/Wasser (7/3) gereinigt werden.
   Ausbeute: 40,4g (57% d.Th.; farblose Kristalle)
   Die Struktur steht im Einklang mit Elementaranalyse, ¹H-NMR- und UV-Spektrum.
b. Herstellung der Endverbindung: 7,15 g (0.02mol) 2-[4,6-Bis-(4-hydroxyphenyl)-s-triazin-2-yl]-phenol werden zusammen mit 80 ml Methylcellosolve und 3,36 g einer 50%-igen Natronlauge (0,044 mol) in einem 250ml Sulfierkolben mit Rührer, Kühler, Innenthermometer und Tropftrichter vorgelegt. Die klare, gelbe Lösung wird auf 60°C erwärmt und nach 15 Minuten Rühren langsam (Tropftrichter) mit 8,76g (0,044 mol) 3-(Brommethyl)-heptan versetzt. Nach 8 Stunden Rühren am Rückfluss (118°C) ist die Alkylierung beendet (Dünnschichtchromatogramm). Es wird unter Vakuum zur Trockene eingedampft, der Rückstand in 100 ml Toluol aufgenommen und zweimal mit Wasser ausgeschüttelt. Nach Trocknung über Natriumsulfat und Abdestillieren des Lösungsmittels erhält man 21,0 g einer gelben Paste. Das Rohprodukt wird in 60ml Toluol/Essigester (95/5) gelöst und zur Reinigung über Kieselgel chromatographiert. Man erhält 7,1 g (61% d.Th.) Produkt als leicht gelbes Harz, welches nach einigen Tagen kristallisiert (Fp. = 56-57°C; farblose Kristalle mit leichtem Gelbstich).
   Die Struktur steht im Einklang mit Elementaranalyse, ¹H-NMR- und UV-Spektrum (λₘₐₓ =
   320nm, εₘₐₓ = 57 000 M⁻¹cm⁻¹).

### Beispiel 19: Die Verbindung der Formel (117) ist auch durch Ringschluß des entsprechenden 4-Alkoxy-benzamidins mit Salicylsäureestem gemäß obigem Verfahren zugänglich.

### Apalikationsbeispiele

**Tabelle 4:**

| | Lichtechtheiten | |
|---|---|---|
| | 4 Perioden DIN 75.202 | 600 kJ nach SAE J 71885 |
| Paste 1 | 1-2 | 1 |
| | | |
| Paste 2 | 4 | 3-4 |
| | | |
| Paste 3 | 3-4 | 3 |

### Beispiel 22: Anwendung für kosmetischen Lichtschutz

Herstellung einer o/w-Emulsion
Phase (A):
   3 g der Verbindung der Formel (112) werden in
   10 g Sesamöl gelöst. Anschliessend werden
   4 g Glycerylstearat,
   1 g Stearinsäure,
   0,5 g Cetylalkohol und
   0,2 g Polysorbat 20
   hinzugegeben und zusammengeschmolzen

### Beispiel 24: Anwendung für kosmetischen Lichtschutz:

1,2 g des UV-Absorbers der Formel (117) und
6,1 g des Phospholipids Phospholipon 90 oder Phospholipon 90H werden zusammen in
70 ml N-Methylpyrrolidon gelöst.
0,33 g Hexadecyltrimethylammoniumchlorid werden zunächst in
190 ml einer Wasser/Ethanol-Mischung (1/10) gelöst und dann zur Lösung von UV-Absorber und Phospholipid zugegeben. Die resultierende Mischung wird zu rende Mischung wird zu 2 I wässriger 0,03%-iger NaCl-Lösung zugetropft, wobei sich unilamellare Vesikel bilden. Die Vesikel-Suspension wird durch Diafiltration auf 100ml eingeengt und das Lösungsmittel gegen wässrige 0,03%-ige NaCl-Lösung ausgetauscht. Anschliessend werden
0,6 g Hydroxycellulose und
0,1 g 2-Brom-2-nitropropan-1,3-thiol zugegeben.

Die Durchmesser der Vesikel werden mit Photonenkorrelationsspektroskopie zu (150±50) nm bestimmt.

Die Formulierung hat einen Sonnenschutzfaktor nach Diffey und Robson von SPF = 6.

## Patentansprüche

1. Hydroxyphenyl-s-triazin der Formel 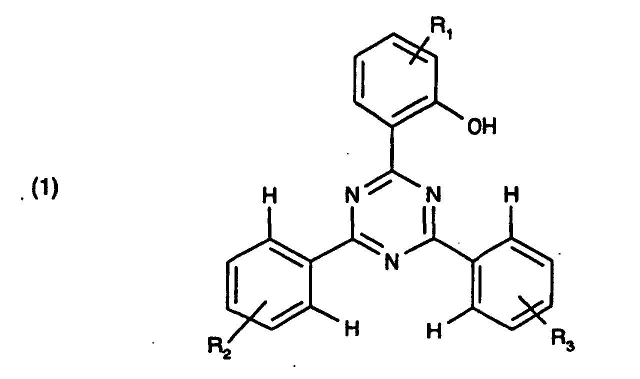 worin
R₁ Wasserstoff; und
R₂ und R₃ 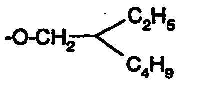 bedeuten.

2. Verfahren zur Herstellung der Hydroxyphenyl-s-triazine nach Anspruch 1, **dadurch gekennzeichnet, dass** man eine Salicylverbindung der Formel 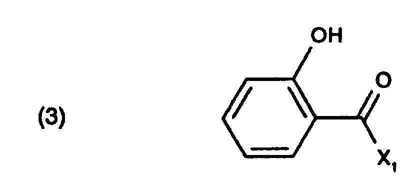 mit einer Benzamidinverbindung der Formel 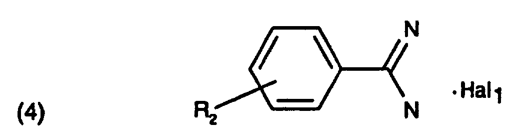 zur Triazinverbindung der Formel (1) umsetzt, wobei R₂ die in Formel (1) angegebene Bedeu
X₁ Halogen oder -OR₄,
R₄ C₁-C₃-Alkyl und
Hal₁ Halogen bedeuten.

3. Verfahren zur Herstellung der Hydroxyphenyl-s-triazine nach Anspruch 1, **dadurch gekennzeichnet, dass** man eine Monochlortriazinverbindung der Formel 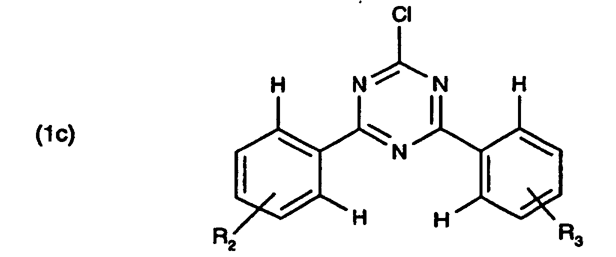 mit einer α-Hydroxyphenylverbindung der Formel 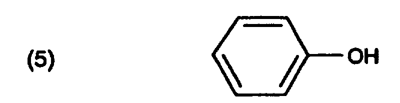 in Gegenwart von Aluminiumchlorid in eine Triazinverbindung der Formel (1) überführt, worin R₂, R₃, und Hal₁, die in Formel (1) angegebene Bedeutung haben.

4. Kosmetisches Präparat enthaltend mindestens eine Verbindung nach Anspruch 1 mit kosmetisch verträglichen Träger- oder Hilfsstoffen.

5. Präparat nach Anspruch 4, **dadurch gekennzeichnet, dass** es weitere UV-Absorber enthält.

6. Präparat nach Anspruch 4 oder 5, **dadurch gekennzeichnet, dass** es als weitere UV-Absorber Oxanilide, Triazole, Vinylgruppen enthaltende Amide oder Zimtsäureamide enthält.

## Claims

1. A hydroxyphenyl-s-triazine of the formula 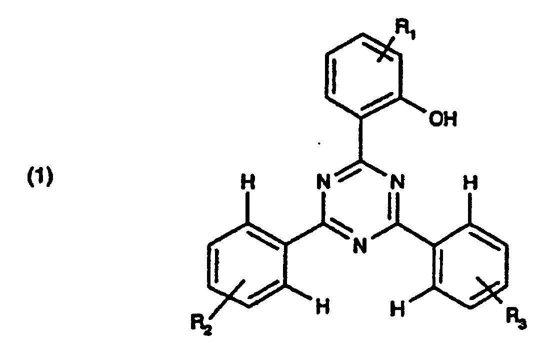 in which
R₁ is hydrogen; and
R₂ and R₃ are

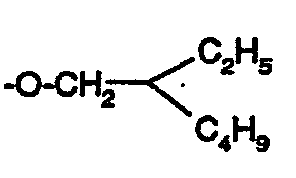

2. A process for preparing a hydroxyphenyl-s-triazine according to claim 1, which comprises reacting a salicylic compound of the formula 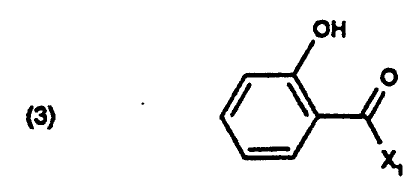 with a benzamidine compound of the formula 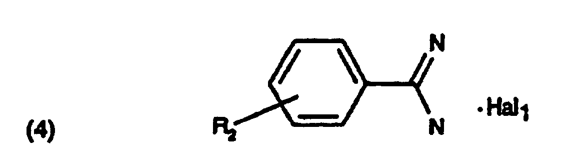 to give the triazine compound of the formula (1) where
R₂ is as defined for formula (1),
X₁ is halogen or -OR₄,
R₄ is C₁-C₃alkyl and
Hal₁ is halogen.

3. A process for preparing a hydroxyphenyl-s-triazine according to claim 1, which comprises reacting a monochlorotriazine compound of the formula 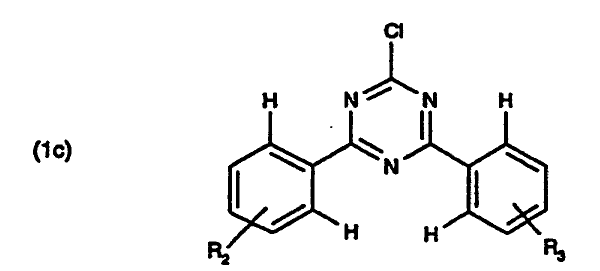 with an α-hydroxyphenyl compound of the formula 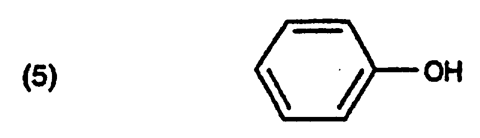 in the presence of aluminium chloride to give a triazine compound of the formula (1) in which
R₂, R₃, and Hal₁ are as defined for formula (1).

4. A cosmetic preparation comprising at least one compound according to claim 1 with cosmetically acceptable excipients or auxiliaries.

5. A preparation according to claim 4, which comprises further UV absorbers.

6. A preparation according to claim 4 or 5, which comprises as further UV absorbers oxanilides, triazoles, vinyl-containing amides or cinnamamides.

## Revendications

1. Hydroxyphényl-s-triazine de formule 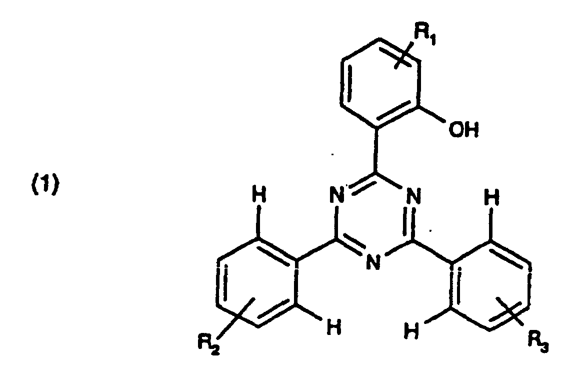 dans laquelle
R₁ représente un atome d'hydrogène ; et
R₂ et R₃ représentent

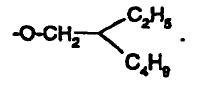

2. Procédé de préparation des hydroxyphényl-s-triazines selon la revendication 1, **caractérisé en ce que** l'on met à réagir un composé salicylique de formule 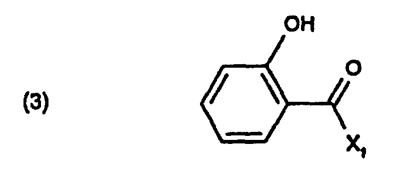 avec un composé benzamidine de formule 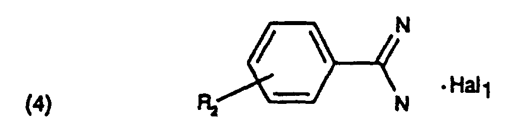 pour obtenir un composé triazine de formule (1), dans lequel
R₂ a la signification indiquée à la formule (1),
X₁ représente un halogène ou -OR₄,
R₄ représente un groupe alkyle en C₁ à C₃ et
Hal₁ représente un halogène.

3. Procédé de préparation des hydroxyphényl-s-triazines selon la revendication 1, **caractérisé en ce que** l'on transforme un composé monochlorotriazine de formule 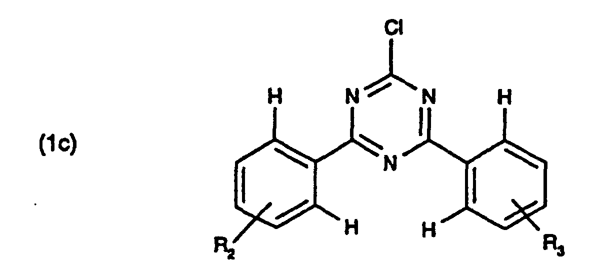 avec un composé α-hydroxyphényle de formule 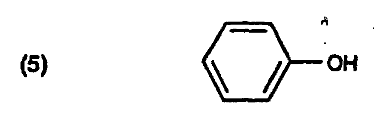 en présence de chlorure d'aluminium en un composé triazine de formule (1), dans laquelle R₂, R₃ et Hal₁ ont les significations indiquées à la formule (1).

4. Préparation cosmétique contenant au moins un composé selon la revendication 1 avec des supports ou adjuvants cosmétiquement acceptables.

5. Préparation selon la revendication 4, **caractérisée en ce qu'**elle contient d'autres absorbants d'UV.

6. Préparation selon la revendication 4 ou 5, **caractérisée en ce qu'**elle contient comme autres absorbants d'UV des oxanilides, des triazoles, des amides contenant des groupes vinyle ou des amides d'acide cinnamique.
